# EUROPÄISCHE PATENTSCHRIFT

(11) **EP 2 426 236 B1**
(45) Veröffentlichungstag und Bekanntmachung des Hinweises auf die Patenterteilung: **02.01.2013**
(21) Anmeldenummer: 10009893.8
(22) Anmeldetag: 20.09.2010
(51) Int. Cl.: C25B 1/12, C25B 15/00, C07C 1/12, C07C 29/151, C07C 31/04

(54) **Verfahren und Energieträger-Erzeugungsanlage zum kohlendioxidneutralen Ausgleich von Erzeugungsspitzen und Erzeugungstälern bei der Erzeugung von elektrischer Energie und/oder zur Erzeugung eines kohlenwasserstoffhaltigen Energieträgers**
Method and fuel generation assembly for the carbon dioxide-neutral compensation of energy peaks and troughs in the generation of electrical energy and/or for producing a fuel containing hydrocarbons
Procédé et installation de production de support d'énergie pour l'équilibrage neutre en dioxyde de carbone de pointes de production et de creux de production lors de la production d'énergie électrique et/ou pour la production d'un support d'énergie contenant de l'hydrocarbure

(30) Priorität: 03.09.2010 EP 10009165
(43) Veröffentlichungstag der Anmeldung: 07.03.2012
(73) Patentinhaber: Carbon-Clean Technologies AG, 50678 Köln (DE)
(72) Erfinder: Knop, Klaus, 79295 Sulzburg (DE); Zoellner, Lars, 50678 Köln (DE)
(74) Vertreter: Gesthuysen, von Rohr & Eggert

(56) Entgegenhaltungen:
- WO-A1-2009/016228
- DE-A1- 4 332 789
- DE-A1-102006 034 712
- US-A1- 2009 289 227

## Beschreibung

Die Erfindung betrifft ein Verfahren und eine Energieträger-Erzeugungsanlage zum kohlendioxidneutralen Ausgleich von Erzeugungsspitzen und Erzeugungstälern bei der Erzeugung von elektrischer Energie, insbesondere aus regenerativen Energiequellen, weiter insbesondere bei der Erzeugung von elektrischer Energie durch Umwandlung von Windenergie und/oder Sonnenenergie und/oder Erdwärme und/oder durch Nutzung von Biomasse und/oder von Gezeitenkräften, und/oder zur Erzeugung eines kohlenwasserstoffhaltigen Energieträgers.

Aus der DE 10 2006 034 712 A1 ist ein Verfahren zur Reduzierung der CO₂-Emission fossilbefeuerter Kraftwerksanlagen bekannt, wobei aus Wasserstoff und im Rauchgas der Kraftwerksanlage enthaltenem Kohlendioxid Kohlenwasserstoffe synthetisiert werden und wobei der Wasserstoff gegebenenfalls unter Einsatz eines Teils der durch die Kraftwerksanlage erzeugten Elektroenergie elektrolytisch gewonnen wird. Das bekannte Verfahren dient dazu, unter Reduzierung der CO₂-Emission Kraftstoffe herzustellen.

Aus der DE 43 32 789 A1 ist ein Verfahren zur Speicherung von Wasserstoffenergie bekannt, wobei eine Mischung von Wasserstoff und Kohlendioxid in einem Reaktor in Methan und/oder Methanol umgesetzt wird. Dabei wird das Kohlendioxid aus dem Abgas fossilbeheizter Energieerzeugungsanlagen verwendet. Methan bzw. Methanol können bei Bedarf als Energieträger für Fahrzeuge, Kraftwerke und Heizanlagen eingesetzt werden.

Aus der US 2009/0289227 A1 ist ein Verfahren zur Herstellung von Methanol aus Kohlendioxid und Wasserstoff bekannt, wobei das Kohlendioxid aus einem Kraftwerksprozess stammen und regenerativ erzeugter Wasserstoff eingesetzt werden kann. Neben Methanol lassen sich mit dem bekannten Verfahren auch Methan, Kohlenmonoxid, Synthesegase und sonstige flüssige Kraftstoffe erzeugen.

Die WO 2009/016228 A1 betrifft eine Elektrolyseeinheit für die Hochtemperatur- und Hochdruckelektrolyse.

Die Erschließung und die Nutzbarmachung erneuerbarer regenerativer Energiequellen, wie beispielsweise der Wind- und Sonnenenergie, aber auch der Erdwärme und der Gezeitenenergie, alternativ oder parallel zur konventionellen Energieerzeugung aus fossilen Energieträgern gewinnt vor dem Hintergrund sich langfristig erschöpfender fossiler Brennstoffe und der Erderwärmung durch Klimagase immer mehr an Bedeutung.

Im Bereich der Nutzung regenerativer Energiequellen zur Stromerzeugung ist von Nachteil, dass die naturgegebene Angebotssituation schwer zu prognostizieren ist und natürlichen Schwankungen unterliegt. Beispielsweise treten aufgrund von witterungs- oder tages- oder jahreszeitbedingten Änderungen entweder Energieerzeugungsspitzen oder aber auch Energieerzeugungstäler der aus der regenerativen Energiequelle erzeugten elektrischen Energie auf. Der schwankenden, insbesondere auf witterungsbedingte oder tages- oder jahreszeitbedingte Einflüsse zurückzuführenden Stromproduktion steht eine nicht konstante Stromnachfrage durch den Verbraucher gegenüber.

Wird Strom aus regenerativen Energiequellen in ein öffentliches Stromnetz eingespeist, können Energieerzeugungsspitzen und Energieerzeugungstäler zu erheblichen Problemen führen, da die Anpassung der Kraftwerkstechnologie an schwankende in das Stromnetz eingespeiste Strommengen nur unter erheblichem Aufwand möglich sind.

Beispielsweise treten bei der Erzeugung von elektrischer Energie aus Windenergie witterungsbedingte Unabwägbarkeiten der Stromproduktion auf, wobei konventionell betriebene Kraftwerke in Spitzenabnahmezeiten bei Windflaute mit Höchstlast gefahren werden, während beispielsweise in Zeiten, in welchen ausreichend Wind zur Stromerzeugung zur Verfügung steht, die Energieabnahmemengen so gering sein können, dass das Kraftwerk mit Unterlast gefahren werden muss, was zu einem höheren Kohlendioxidausstoß führt. Wenn darüber hinaus Betreiber von Windkraftanlagen den Strom aufgrund einer drohenden Netzüberlastung nicht in das Stromnetz einspeisen können, ist eine Abschaltung der Windkraftanlage geboten, was zu einer Abnahme der Wirtschaftlichkeit beim Betrieb der Windkraftanlage führt.

Im Bereich der Nutzung regenerativer Energiequellen zur Stromerzeugung besteht daher ein Bedarf nach einem wirtschaftlichen und effizienten Verfahren zum Ausgleich von Erzeugungsspitzen und Erzeugungstälern.

Aus dem Stand der Technik ist bereits bekannt, mit Hilfe einer Elektrolyse Wasser in Wasserstoff und Sauerstoff aufzuspalten. Die Wasserstoffspeicherung ist jedoch verfahrenstechnisch aufwendig und kostenintensiv. Darüber hinaus erfordert der Transport des Wasserstoffs an einen Verbrauchsort eine Wasserstoffinfrastruktur, die in der Regel nicht vorhanden ist. Weiterhin ist es von Nachteil, dass der bei der Elektrolyse freigesetzte Sauerstoff nur eingeschränkt einer Verwertung zugeführt wird. Daher wird im Stand der Technik bereits vorgesehen, Kohlendioxid und Wasserstoff unter Verwendung von Katalysatoren in einen kohlenwasserstoffhaltigen Energieträger umzuwandeln, beispielsweise in Methanol oder in Methan. Verfahren zum Bereitstellen speicherbarer und transportabler kohlenstoffbasierter Energieträger unter Einsatz von Kohlenstoffdioxid als Kohlenstofflieferant und unter Einsatz von elektrischer Energie sind in der WO 2010/069622 A1 beschrieben. Die katalytische Methanolherstellung wird darüber hinaus in der WO 20101069385 A1 und in der WO 2010/069685 A1 offenbart.

Aufgabe der vorliegenden Erfindung ist es, ein Verfahren und eine Energieträger-Erzeugungsanlage der eingangs genannten Art zur Verfügung zu stellen, die bei hoher Wirtschaftlichkeit und hohem Wirkungsgrad den Ausgleich von Erzeugungsspitzen und Erzeugungstälern bei der Erzeugung von elektrischer Energie, insbesondere aus regenerativen Energiequellen, ermöglichen und/oder die Erzeugung eines kohlenwasserstoffhaltigen Energieträgers zulassen, wobei der Ausgleich von Schwankungen der erzeugten bzw. anfallenden Energiemengen einerseits und die Erzeugung des Energieträgers andererseits gleichermaßen im Wesentlichen kohlendioxidneutral, das heißt im Wesentlichen ohne Freisetzung von Kohlendioxid, erfolgen sollen.

Die vorgenannte Aufgabe wird durch ein Verfahren mit den Merkmalen des Patentanspruchs 1 und durch eine Energieträger-Erzeugungsanlage mit den Merkmalen des Patentanspruchs 8 gelöst.

Erfindungsgemäß wird aus einer Energiequelle erzeugte elektrische Energie in einer Elektrolyseeinheit zur Wasserstofferzeugung durch Elektrolyse eines wässrigen Mediums, insbesondere von Wasser, eingesetzt, wobei ein in der Elektrolyseeinheit erzeugter Wasserstoffstrom einer Reaktoreinheit ausgebildet zur insbesondere katalytischen Erzeugung eines kohlenwasserstoffhaltigen Energieträgerstroms unter Einsatz von Wasserstoff und Kohlendioxid, insbesondere ausgebildet zur katalytischen Erzeugung von Methanol oder Methan, zugeführt wird, wobei der erzeugte kohlenwasserstoffhaltige Energieträgerstrom zumindest teilweise und bedarfsabhängig in einer Brennkammer verbrannt und die thermische Energie des bei der Verbrennung gebildeten Rauchgasstroms zur Erzeugung von elektrischer Energie in einem Gasturbinenprozess und/oder in einem Dampfturbinenprozess genutzt wird und wobei der Rauchgasstrom der Reaktoreinheit als Kohlenstoffquelle für die Erzeugung des kohlenwasserstoffhaltigen Energieträgerstroms zugeführt wird.

Der Erfindung liegt der Grundgedanke zugrunde, elektrische Energie, die nicht regelmäßig anfällt und/oder bei wechselnden Energiemengen, in eine Elektrolyseeinheit einzuspeisen, um mittels der eingespeisten elektrischen Energie Wasserstoff zu erzeugen, der anschließend in einer Reaktoreinheit insbesondere katalytisch unter Verwendung von Kohlendioxid in einen kohlenwasserstoffhaltigen Energieträger, vorzugsweise in Methanol oder Methan, umgewandelt wird. Durch spätere teilweise und bedarfsabhängige Verbrennung des erzeugten kohlenwasserstoffhaltigen Energieträgerstroms in einer Brennkammer der Energieträger-Erzeugungsanlage wird ein heißer Rauchgasstrom freigesetzt, der in einem nachgeschalteten Gasturbinenprozess und/oder in einem Dampfturbinenprozess genutzt werden kann zur Stromerzeugung. Durch Rückspeisung des erzeugten Stroms ins Stromnetz lassen sich Erzeugungsspitzen und Erzeugungstäler bei der Erzeugung von elektrischer Energie, insbesondere aus regenerativen Energiequellen, weiter insbesondere bei der Erzeugung von elektrischer Energie mittels Windkraftanlagen und/oder Photovoltaikanlagen, in Form einer konstanten Stromabgabe über eine lange Zeit ausgleichen. Das erfindungsgemäße Verfahren und die erfindungsgemäße Energieträger-Erzeugungsanlage eignen sich dabei insbesondere zum Ausgleich von Schwankungen bei der Stromproduktion in Windparks. Darüber hinaus führt das erfindungsgemäße Verfahren zu einer Verringerung des Bedarfs an Reserveenergiekraftwerken, da Strom in Form des Energieträgerstroms zwischengespeichert wird.

Mit dem erfindungsgemäßen Verfahren und der erfindungsgemäßen Energieträger-Erzeugungsanlage lassen sich größere oder kleinere angeforderte Strommengen kurzfristig bereitstellen. Für einen kohlendioxidneutralen Ausgleich von Erzeugungsspitzen und Erzeugungstälern ist dabei erfindungsgemäß vorgesehen, dass der in der Brennkammer erzeugte Rauchgasstrom der Reaktoreinheit als Kohlenstoffquelle für die Erzeugung des kohlenwasserstoffhaltigen Energieträgerstroms zugeführt wird. Es wird damit ein geschlossener Kohlendioxidkreislauf erhalten, was zu einer Entlastung der Umwelt durch eingesparte Kohlendioxidemissionen führt. Gleichzeitig wird mit der Erfindung ein Energiespeicher in Form eines kohlenwasserstoffhaltigen Energieträgerstroms zur Verfügung gestellt, der es ermöglicht, bei hohem Strombedarf den gespeicherten kohlenwasserstoffhaltigen Energieträger wieder in einem Kraftwerksprozess zur Stromerzeugung einzusetzen und den erzeugten Strom in ein Stromnetz einzuspeisen.

Ein Teil der im Gasturbinenprozess und/oder im Dampfturbinenprozess erzeugten elektrischen Energie kann zur Eigennutzung in der erfindungsgemäßen Energieträger-Erzeugungsanlage vorgesehen sein, beispielsweise zur Verdichtung/Kompression von Stoffströmen und/oder für die Elektrolyse von Wasser zur Erzeugung des Wasserstoffstroms in der Elektrolyseeinheit.

Grundsätzlich ist es möglich, dass ein Teil des erzeugten Energieträgers verbrannt wird zur Stromerzeugung vor Ort, während ein weiterer Teil für eine externe Verwertung bereitgestellt wird. Der erzeugte Energieträger kann als Produkt veräußert und außerhalb der Energieträger-Erzeugungsanlage eingesetzt werden, beispielsweise als Benzinersatz und/oder Rohstoff in der chemischen Industrie.

Darüber hinaus kann der Brennkammer wenigstens ein weiterer (externer) kohlenwasserstoffhaltiger Energieträgerstrom, insbesondere Erdgas, zugeführt werden. Dadurch lässt sich gleichermaßen Kohlendioxid für die Erzeugung des kohlenwasserstoffhaltigen Energieträgers in der Reaktoreinheit bereitstellen und elektrischer Strom erzeugen durch Nutzung des bei der Verbrennung des externen Energieträgerstroms in der Brennkammer gebildeten Rauchgasstroms in dem Gasturbinenprozess und/oder dem Dampfturbinenprozess. Dies ist insbesondere dann von Vorteil, wenn die erfindungsgemäße Energieträger-Erzeugungsanlage ausschließlich zur Produktion des Energieträgers in der Reaktoreinheit eingesetzt wird und, weiter insbesondere, wenn die zur Verfügung stehende elektrische Energie aus einer regenerativen Energiequelle nicht ausreicht, um ausreichend Strom für die Elektrolyseeinheit zu liefern.

Bei niedrigen Strompreisen kann auch Strom aus fossilen Energiequellen in die Elektrolyseeinheit eingespeist werden, um kostengünstig einen kohlenwasserstoffhaltigen Energieträger in der Reaktoreinheit zu erzeugen, der für eine Verstromung zur Verfügung steht, wenn die Strompreise wieder gestiegen sind. Falls beispielsweise kurzfristig keine Wind- oder Sonnenenergie zur Verfügung steht, kann über die Nutzung von Erdgas konventionell erzeugter Strom in die Elektrolyse eingespeist werden, was aufgrund des hohen Wirkungsgrades der erfindungsgemäßen Energieträger-Erzeugungsanlage eine preisgünstige und klimaneutrale, d. h. kohlendioxidausstoßfreie, Erzeugung des Energieträgers in der Reaktoreinheit zulässt. Hierdurch lässt sich eine hohe Wirtschaftlichkeit des erfindungsgemäßen Verfahrens und der erfindungsgemäßen Energieträger-Erzeugungsanlage gewährleisten, wobei grundsätzlich auch Mischstrom aus erneuerbarer und fossiler Energie zur Elektrolyse eingesetzt werden kann. Die zuvor beschriebene Verfahrensführung bzw. Betriebsweise der erfindungsgemäßen Energieträger-Erzeugungsanlage ist insbesondere im Zusammenhang mit jahreszeitlich oder tageszeitlich bedingten Strompreisänderungen (Sommer-Winter-Zyklus; Tag-Nacht-Zyklus) von Vorteil.

Im Übrigen kann der durch Elektrolyse erzeugte Wasserstoffstrom mit einem weiteren Wasserstoffstrom, beispielsweise aus der Biomassevergärung und/oder -vergasung, vermischt werden, um den der Reaktoreinheit zugeführten Wasserstoffvolumenstrom zu vergleichmäßigen oder zu vergrößern. Dadurch lässt sich beispielsweise die Nutzung von Biomasse zur regenerativen Stromerzeugung in das Anlagenkonzept der erfindungsgemäßen Energieträger-Erzeugungsanlage integrieren. Um eine hohe Wasserstoffreinheit des weiteren, nicht durch Elektrolyse erzeugten Wasserstoffstroms zu gewährleisten, kann eine entsprechend ausgebildete Gasreinigungsanlage vorgeschaltet sein. Der weitere Wasserstoffstrom kann auch direkt der Reaktoreinheit zugeführt werden.

Durch den Einsatz von Sauerstoff an der Stelle von Luft kann die Gewinnung von fast reinem Kohlendioxid als Rauchgasstrom erreicht werden, wobei erfindungsgemäß dementsprechend vorgesehen sein kann, dass der Brennkammer ein in der Elektrolyseeinheit erzeugter Sauerstoffstrom zur Verbrennung des Energieträgerstroms zugeführt wird.

Zur Erzeugung des Wasserstoffstroms und des Sauerstoffstroms kann eine Druck-Elektrolyseeinheit vorgesehen sein, wobei der Wasserstoffdruck und/oder der Sauerstoffdruck zwischen 10 bis 200 bar, vorzugsweise zwischen 30 bis 100 bar, insbesondere wenigstens 60 bis 80 bar, betragen kann. Aus dem Stand der Technik sind hierfür geeignete und dem Fachmann bekannte alkalische und Polymerelektrolytmembran-Druckelektrolyseverfahren bekannt, wobei mit Druck-Elektrolyseeinheiten zukünftig auch ein Systemdruck von mehr als 200 bar erreicht werden kann

Der unter Druck stehende Wasserstoff kann einem Druckspeicher als Pufferbehälter zugeführt und dort gespeichert werden. Der Sauerstoffstrom kann mit einem Speicherdruck von wenigstens 30 bar, insbesondere ca. 60 bar oder mehr, gespeichert werden. Anschließend kann dann ein Sauerstoffstrom mit einem Speicherdruck von wenigstens 30 bar, insbesondere ca. 60 bar oder mehr, vorzugsweise ohne Zwischenverdichtung der Brennkammer zugeführt werden. Durch Einsatz einer Druck-Elektrolyseeinheit sinkt somit der Verfahrensaufwand bei der Stromproduktion im Gasturbinenprozess erheblich, da kein Verdichter zwischen dem Sauerstoffspeicher und der Brennkammer vorgesehen sein muss. Dies führt zu einer erheblichen Kostenreduzierung und Wirkungsgradsteigerung.

Durch Speicherung des Wasserstoffstroms und/oder des Sauerstoffstroms und/oder des in der Reaktoreinheit erzeugten Energieträgerstroms in entsprechend ausgebildeten Speichereinheiten ist es möglich, die vorgenannten Ströme bedarfsabhängig zu verbrauchen und/oder für eine Verwertung als Rohstoff bzw. Energielieferant außerhalb der erfindungsgemäßen Energieträger-Erzeugungsanlage vorzusehen. Durch die Speicherung des erzeugten Energieträgerstroms lässt sich bedarfsweise eine größere oder eine kleinere Menge des Energieträgerstroms zur Stromerzeugung in der erfindungsgemäßen Energieträger-Erzeugungsanlage einsetzen, um eine gleichbleibend große Strommenge bereitzustellen und eine optimale Nutzung erneuerbarer Energien zur Stromerzeugung zu gewährleisten.

Darüber hinaus ist es auch möglich, Kohlendioxid aus dem Rauchgas und/oder einen externen Kohlendioxidstrom in einer Speichereinheit zu speichern und der Reaktoreinheit bedarfsabhängig zuzuführen. Zur Speicherung kann der Kohlendioxidstrom verdichtet, vorzugsweise verflüssigt, werden. Durch die Verbrennung des Energieträgerstroms mit reinem Sauerstoff lässt sich ein Verbrennungsabgas erzeugen, das reines Kohlendioxid und Wasserdampf enthält. Dies ermöglicht die Gewinnung von hochreinem Kohlendioxid durch Abtrennung des Wasserdampfs.

Ein aus der Reaktoreinheit und/oder aus dem Rauchgas bei dessen Abkühlung abgeschiedener Kondensatstrom und/oder ein Kühlwasserstrom aus der Reaktoreinheit kann vorzugsweise zur Brennkammer geführt werden, um in einfacher Weise eine Temperaturkontrolle der Verbrennungsreaktionen in der Brennkammer zu ermöglichen. Durch Einbringen des Kondensatstroms aus der Reaktoreinheit, beispielsweise eines Destillatstroms aus einer Methanolanlage, in die Brennkammer wird die Rauchgastemperatur erniedrigt, so dass in einfacher Weise eine Kontrolle bzw. Steuerung der Rauchgastemperatur möglich ist. Zudem kann der Kondensatstrom aus der Reaktoreinheit und/oder aus dem Rauchgas höhere Kohlenwasserstoffe aufweisen, die in der Brennkammer umgesetzt bzw. verbrannt werden. Dadurch wird eine Wasseraufbereitung entbehrlich, was zu einer Verfahrensvereinfachung führt und zu einer hohen Wirtschaftlichkeit beiträgt, Alternativ oder ergänzend kann vorgesehen sein, dass ein Kondensatstrom aus der Reaktoreinheit und/oder ein aus dem Rauchgasstrom abgeschiedener Kondensatstrom als Speisewasser für die Elektrolyseeinheit genutzt wird.

Zur Abwärmenutzung kann vorgesehen sein, die bei der Erzeugung des Energieträgerstroms in der Reaktoreinheit frei werdende Reaktionswärme aus der Reaktoreinheit auszukoppeln. Die Reaktionswärme kann vorteilhaft zur Vor- oder Nachwärmung der betrachteten Stoffströme dienen. Auch eine Femwärmenutzung ist möglich. Beispielsweise sind die an der katalytischen Bildung von Methanol oder Methan aus Kohlendioxid und Wasserstoff beteiligten Reaktionen exotherm, so dass hier eine Abnutzung der Reaktionswärme möglich ist. Darüber hinaus steht Abwärme aus dem Elektrolyseprozess zur Verfügung, die beispielsweise in ein Fernwärmenetz eingespeist werden kann.

Die erfindungsgemäße Energieträger-Erzeugungsanlage weist wenigstens eine Elektrolyseeinheit, wenigstens eine Reaktoreinheit und wenigstens eine Brennkammer auf und ist zur Durchführung des zuvor beschriebenen Verfahrens ausgebildet. Darüber hinaus kann wenigstens eine Gasturbine und/oder wenigstens eine Dampfturbine vorgesehen sein, um den Gasturbinenprozess bzw. den Dampfturbinenprozess ausführen zu können. Schließlich können Speichereinheiten zur Speicherung des in der Elektrolyseeinheit erzeugten Wasserstoffs und/oder des Sauerstoffs und/oder des in der Reaktoreinheit erzeugten Energieträgers und/oder des aus dem Rauchgasstrom abgeschiedenen Kohlendioxids vorgesehen sein. Wärmetauscher, Verdichter und entsprechende Leitungsführungen sind ebenfalls Bestandteile der Energieträger-Erzeugungsanlage, wobei die vorgenannte Aufzählung nicht abschließend ist.

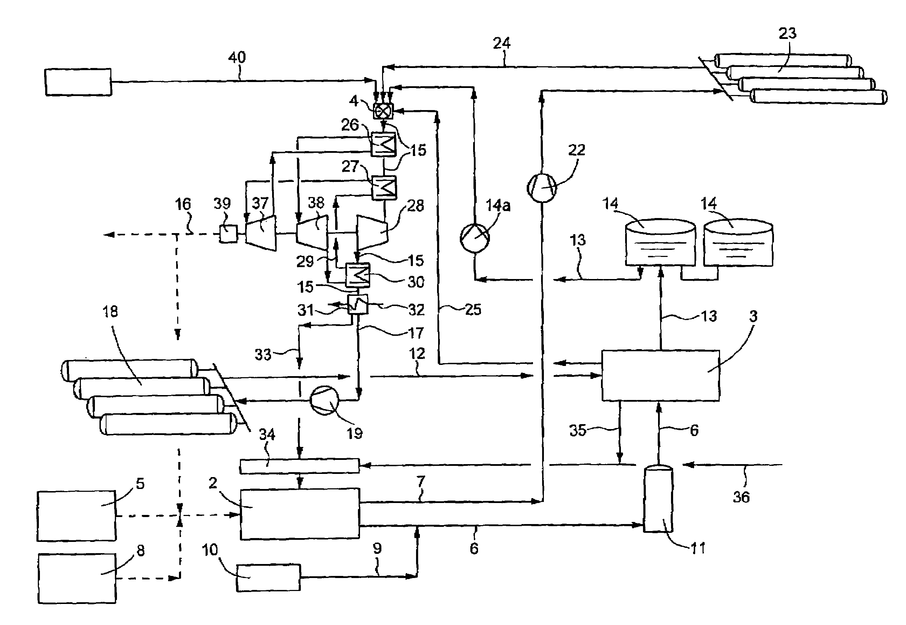

Im Einzelnen gibt es eine Vielzahl von Möglichkeiten, das erfindungsgemäße Verfahren und die erfindungsgemäße Energieträger-Erzeugungsanlage auszugestalten und weiterzubilden, wobei einerseits auf die abhängigen Patentansprüche und andererseits auf die nachfolgende detaillierte Beschreibung eines bevorzugten Ausführungsbeispiels der Erfindung unter Bezugnahme auf die Zeichnung verwiesen wird.

In der einzigen Figur der Zeichnung ist schematisch ein Verfahren zum kohlendioxidneutralen Ausgleich von Erzeugungsspitzen und Erzeugungstälern bei der Erzeugung von elektrischer Energie aus regenerativen Energiequellen, beispielsweise aus Windenergie- und/oder Photovoltaikanlagen, dargestellt. Zur Ausführung des Verfahrens ist eine Energieträger-Erzeugungsanlage 1 vorgesehen.

Die Anlage 1 weist eine Elektrolyseeinheit 2, eine Reaktoreinheit 3 und eine Brennkammer 4 auf. In der Elektrolyseeinheit 2 wird nicht regelmäßig anfallender oder in schwankenden Mengen verfügbarer elektrischer Strom 5, beispielsweise aus Windkraftanlagen oder Photovoltaik-Anlagen, zur Erzeugung eines Wasserstoffstroms 6 und eines Sauerstoffstroms 7 eingesetzt. Vorzugsweise wird Wasserstoff und Sauerstoff mittels Druckelektrolyse erzeugt, was gegenüber der atmosphärischen Elektrolyse den Vorteil bietet, dass die Produktgase Wasserstoff und Sauerstoff mit einem Druck vorzugsweise zwischen 30 bis 80 bar, insbesondere ca. 60 bar, zur Verfügung stehen.

Alternativ oder ergänzend zum Strom 5 aus regenerativen Energiequellen kann vorgesehen sein, (kostengünstigen) Strom 8 aus anderen, insbesondere fossilen Energiequellen, zur Elektrolyse einzusetzen.

In einem weiteren Verfahrensschritt wird der unter Druck stehende Wasserstoffstrom 6, gegebenenfalls vermischt mit einem weiteren Wasserstoffstrom 9 aus einer Biogasanlage 10, einem Pufferbehälter als Speichereinheit 11 zugeführt und anschließend mit einem Kohlendioxidstrom 12 in der Reaktoreinheit 3 katalytisch zu Methanol als Energieträger umgesetzt. Aus der Reaktoreinheit 3 tritt ein Methanolstrom als kohlenwasserstoffhaltiger Energieträgerstrom 13 aus, der in Methanoltanks als weitere Speichereinheiten 14 zwischengespeichert wird.

Der erzeugte kohlenwasserstoffhaltige Energieträgerstrom 13 kann dann zumindest teilweise und bedarfsabhängig in der Brennkammer 4 verbrannt werden. Der Energieträgerstrom 13 wird dazu über eine Pumpe 14a der Brennkammer 4 zugeführt.

Die thermische Energie des bei der Verbrennung gebildeten Rauchgasstroms 15 wird zur Erzeugung von elektrischem Strom 16 in einem gekoppelten Gas- und Dampfturbinenprozess genutzt, wobei der Rauchgasstrom 15 als Kohlenstoffquelle für die Erzeugung des kohlenwasserstoffhaltigen Energieträgerstroms 13 dienen kann. Zu diesem Zweck wird aus dem Rauchgasstrom 15 ein Kohlendioxidstrom 17 abgetrennt, Hierbei lässt es die Erfindung zu, dass der Kohlendioxidstrom 17 in einer weiteren Speichereinheit 18 zwischengespeichert und bedarfsabhängig zur Reaktoreinheit 3 geführt wird. Vorzugsweise wird der Kohlendioxidstrom 17 mit einem Verdichter 19 verflüssigt und unter Druck gespeichert, so dass Kohlendioxid unter Druck dem Methanolprozess zugeführt werden kann, um mit dem Wasserstoffstrom 6 aus der Elektrolyseeinheit 2 zu Methanol umgesetzt zu werden.

Der Sauerstoffstrom 7 aus der Elektrolyseeinheit 2 wird mit einem Verdichter 22 verdichtet, einer Sauerstoff Speichereinheit 23 zugeführt und dort zwischengespeichert, Aus der weiteren Speichereinheit 23 wird dann ein Sauerstoffstrom 24 zusammen mit dem Energieträgerstrom 13 der Brennkammer 4 zugeführt, wobei in der Brennkammer 4 der Energieträger, im vorliegenden Fall Methanol, stöchiometrisch zu Wasser und Kohlendioxid umgesetzt wird. Durch die Verbrennung mit reinem Sauerstoff wird die Gewinnung von fast reinem Kohlendioxid als Rauchgasstrom 15 erreicht.

Da die Verbrennung mit reinem Sauerstoff zu hohen Verbrennungstemperaturen führt, ist bei der gezeigten Anlage 1 weiter vorgesehen, dass ein überhitzter Wasserstrom 25, bei dem es sich vorzugsweise um Kühlwasser oder mit höheren Kohlenwasserstoffen belastetes Kondensat aus der Methanolanlage handelt, in die Brennkammer 4 eingebracht, vorzugsweise eingedüst, wird. Dadurch ist eine einfache Führung und Steuerung des Verbrennungsprozesses möglich.

Das die Brennkammer 4 verlassende Rauchgas 15 wird über zwei Wärmetauscher 26, 27 abgekühlt und über eine Gasturbine 28 entspannt. Nach der Entspannung wird das Rauchgas 15 zur Erzeugung eines Sattdampfstroms 29 in einem weiteren Wärmetauscher 30 genutzt und schließlich in einem Abscheider 31 mit einem externen Kühlmittelstrom 32 so abgekühlt, dass im Wesentlichen Kohlendioxid als Gasphase austritt und den Kohlendioxidstrom 17 bildet.

Ein aus dem Abscheider 31 austretender Kondensatstrom 33 kann einem Wasserreservoir 34 der Elektrolyseeinheit 2 zugeführt werden. Alternativ oder ergänzend kann ein Kondensatstrom 35 aus der Reaktoreinheit 3 dem Wasserreservoir 34 zugeführt werden. Alternativ oder ergänzend kann zudem ein Reinwasserstrom 36 zur Wasserversorgung der Elektrolyseeinheit 2 vorgesehen sein.

Der Sattdampfstrom 29 wird in dem Wärmetauscher 27 überhitzt und einer ersten Stufe 37 einer Dampfturbine zugeführt. Die Dampfturbine kann vorzugsweise mit Zwischenüberhitzung arbeiten, wobei die Zwischenüberhitzung in dem Wärmetauscher 26 erfolgt. Entsprechend ist eine zweite Stufe 38 der Dampfturbine vorgesehen. Die Erzeugung des elektrischen Stroms 16 erfolgt in an sich bekannter Weise mit einer Generatoreinheit 39. Der erzeugte Strom 16 kann entweder für die Elektrolyse von Wasser in der Elektrolyseeinheit 2 genutzt oder in ein Stromnetz eingespeist werden, um Erzeugungstäler bei der Erzeugung von elektrischem Strom 5 aus regenerativen Energiequellen auszugleichen.

Wie sich aus der Zeichnung weiter ergibt, ist eine Aufbereitung von Abwasser bei der gezeigten Anlage 1 nicht zwingend erforderlich, wenn der Kondensatstrom 33 aus dem Abscheider 31 und der Kondensatstrom 35 aus der Reaktoreinheit 3 der Elektrolyseeinheit 2 zugeführt werden. Wie beschrieben, können Abwässer aus dem Abscheider 31 und/oder der Reaktoreinheit 3 auch in der Brennkammer 4 umgesetzt werden, was ebenfalls eine Abwasseraufbereitung entbehrlich macht.

Neben dem in der Reaktoreinheit 3 erzeugten Energieträger können der Brennkammer 4 auch andere gasförmige oder flüssige Brennstoffe zugeführt werden, so dass die gezeigte Anlage 1 grundsätzlich auch zur Produktion des Energieträgers ausschließlich für die externe Verwertung genutzt werden kann. In diesem Fall wird der gesamte in der Reaktoreinheit 3 erzeugte Energieträgerstrom 13 in den Speichereinheiten 14 gespeichert und als Energieträgerstrom 21 aus der Anlage 1 abgeführt. Beispielsweise kann ein Erdgasstrom 40 als Kohlenstoffquelle in der Brennkammer 4 zusammen mit dem Sauerstoffstrom 24 verbrannt werden, wobei das dabei frei werdende Kohlendioxid der Reaktoreinheit 3 zugeführt wird. Gleichzeitig wird elektrischer Strom 16 erzeugt, der für die Elektrolyse von Wasser zumindest teilweise genutzt werden kann.

Die dargestellte Anlage 1 ermöglicht somit sowohl im Kraftwerksbetrieb, d. h. bei zumindest teilweiser Verbrennung des in der Reaktoreinheit 3 erzeugten Energieträgerstroms 13, als auch im Produktionsbetrieb, wenn der erzeugte Energieträger vollständig einer externen Nutzung zugeführt wird, eine kohlendioxidneutrale Verfahrensführung, bei der kein Kohlendioxid an die Umwelt abgegeben wird. Ebenso wenig werden Stickoxide oder Schwefeloxide an die Umwelt abgegeben. Schwankungen in der Zuführung von Strom 5 aus regenerativen Energiequellen zur Anlage 1 können durch die Verstromung des Energieträgerstroms 13 oder durch die Verstromung eines externen (fossilen) Energieträgers, beispielsweise durch Zuführung von Erdgas, ausgeglichen werden, so dass eine konstante Stromabgabe an ein Stromnetz möglich ist. Je nach Größe der Speichereinheiten 11, 14, 18, 23 lassen sich Schwankungen in der Bereitstellung erneuerbarer Energie über lange Zeit in Form von konstanter Stromabgabe ausgleichen und größere oder kleinere angeforderte Strommengen kurzfristig bereitstellen oder reduzieren. Gegebenenfalls arbeitet die Anlage 1 auch als Kohlendioxidsenke, wobei Kohlendioxid aus externen Quellen als Kohlendioxidstrom 20 eingespeist werden kann.

Zudem kann die Abwärme aus der Elektrolyse und die Abwärme aus dem Gas- und Dampfprozess in ein Fernwärmenetz eingespeist oder auch direkt zur Vorwärmung von Stoffströmen in der Anlage 1 genutzt werden.

Insbesondere ist es durch die Verbrennung mit reinem Sauerstoff möglich, einen sehr reinen Kohlendioxidstrom zu gewinnen, ohne dass dazu die Gasphase aus dem Abscheider 31 einer chemischen und/oder physikalischen weiteren Behandlung, wie beispielsweise einer Kohlendioxidwäsche, unterzogen werden muss.

Es versteht sich, dass die zuvor beschriebenen Merkmale lediglich eine bevorzugte Ausführungsform der Energieträger-Erzeugungsanlage 1 kennzeichnen und nicht zwingend in der dargestellten Kombination vorgesehen sein müssen. Alternative Kombinationen der beschriebenen Merkmale sind möglich, auch wenn diese nicht im Einzelnen beschrieben ist.

Beispielhafte Betriebszustände der beschriebenen Anlage 1 werden nachfolgend erläutert:
Betrieb bei ausgeglichener Bilanz: Es werden 7220 kW elektrische Energie in die Elektrolyse eingespeist. Es werden 1680 Nm³/h H₂ und 840 Nm³/h O₂ erzeugt, Die erzeugte MeOH-Menge (Methanolmenge) beträgt 0,8 t/h. Diese Menge wird in die Brennkammer eingespeist zusammen mit 840 Nm³/h O₂. Es werden 4200 kW erzeugt. Der GuD-Prozess gibt 560 Nm³/h CO₂ ab.

Bei diesem Betrieb erfolgt keine Entnahme oder Zufuhr von in den Speichern 11, 14, 18, 23 gespeicherten Medien. Der Wirkungsgrad- bezogen auf die eingespeiste/abgegebene Strommenge- beträgt ca. 60 %.

Betrieb bei Stromüberschuss: Es werden z.B. 14440 kW in die Elektrolyse eingespeist. Es werden 3360 Nm³/h H₂ und 1680 Nm³/h O₂ erzeugt. Die erzeugte MeOH-Menge beträgt 1,6 t/h. Von dieser Menge werden 0,8 t/h in die Brennkammer eingespeist zusammen mit 840 Nm³/h O₂. Es werden 4200 kW erzeugt. Der GuD-Prozess gibt 560 Nm³/h CO₂ ab.

Bei diesem Betrieb werden die Methanol-Speicher 14 und der Sauerstoff-Speicher 23 aufgefüllt und der Kohlendioxid-Speicher 18 entladen.

Der Wirkungsgrad- bezogen auf die eingespeiste/abgegebene Strommenge - beträgt ca. 30 %. Zusätzlich werden 0,8 t MeOH/h erzeugt.

Betrieb bei Stromunterschuss: Es werden z.B. 3610 kW in die Elektrolyse eingespeist. Es werden 840 Nm³/h H₂ und 420 Nm³/h O₂ erzeugt. Die erzeugte MeOH-Menge beträgt 1,6 t/h. Von dieser Menge werden 0,8 t/h in die Brennkammer 4 eingespeist zusammen mit 840 Nm³/h O₂. Es werden 4200 kW erzeugt. Der GuD-Prozess gibt 560 Nm³/h CO₂ ab.

Bei diesem Betrieb werden die Methanol-Speicher 14 und der Sauerstoff-Speicher 23 geleert und der Kohlendioxid-Speicher 18 aufgeladen.

Der Wirkungsgrad- bezogen auf die eingespeiste/abgegebene Strommenge - beträgt ca. 116 %. Es werden 0,4 t MeOH/h verbraucht.

Für die gleichzeitige Erzeugung von Strom und Methanol kann ein Erdgasbetrieb der Anlage 1 vorgesehen sein. Es erfolgt dann keine Verbrennung von Methanol:
Betrieb bei Verbrennung von Erdgas: Es werden 7220 kW in die Elektrolyse eingespeist. Es werden 1680 Nm³/h H₂ und 840 Nm³/h O₂ erzeugt. Die erzeugte MeOH-Menge beträgt 0,8 t/h. Diese Menge wird gespeichert. Mit 840 Nm³/h O₂ und 3,58 Gcal/h Erdgas (als CH₄ gerechnet = 4160 kW chemisch) werden 3950 kW erzeugt. Der GuD-Prozess gibt 420 Nm³/h CO₂ ab.

Bei diesem Betrieb werden aus dem Kohlendioxid-Speicher 18 ca. 140 kg/h CO₂ entnommen, der Sauerstoff-Speicher 23 wird weder geladen noch entladen und den Methanol-Speichern 14 werden 0,8 t MeOH zugeführt.

## Patentansprüche

1. Verfahren zum Kohlendioxid neutralen Ausgleich von Erzeugungsspitzen und Erzeugungstälern bei der Erzeugung von elektrischer Energie und/oder zur Erzeugung eines kohlenwasserstoffhaltigen Energieträgers, jeweils unter Verwendung einer Energieträger-Erzeugungsanlage (1), wobei aus einer Energiequelle erzeugte elektrische Energie in einer Elektrolyseeinheit (2) der Energieträger-Erzeugungsanlage (1) zur Wasserstofferzeugung durch Elektrolyse eines wässrigen Mediums eingesetzt wird, wobei ein in der Elektrolyseeinheit (2) erzeugter Wasserstoffstrom (6) einer Reaktoreinheit (3) der Energieträger-Erzeugungsanlage (1) ausgebildet zur Erzeugung eines kohlenwasserstoffhaltigen Energieträgerstroms (13) unter Einsatz von Wasserstoff und Kohlendioxid zugeführt wird, wobei der erzeugte kohlenwasserstoffhaltige Energieträgerstrom (13) zumindest teilweise und bedarfsabhängig mit einem in der Elektrolyseeinheit (2) erzeugten Sauerstoffstrom (7, 24) in einer Brennkammer (4) der Energieträger-Erzeugungsanlage (1) verbrannt und die thermische Energie des bei der Verbrennung gebildeten Rauchgasstroms (15) zur Erzeugung von elektrischer Energie in einem Gasturbinenprozess und/oder in einem Dampfturbinenprozess genutzt wird und der Reaktoreinheit (3) lediglich der gebildete Rauchgasstrom (15) als Kohlenstoffquelle für die Erzeugung des kohlenwasserstoffhaltigen Energieträgerstroms (13) zugeführt wird.

2. Verfahren nach Anspruch 1, **dadurch gekennzeichnet, dass** der Brennkammer (4) wenigstens ein weiterer kohlenwasserstoffhaltiger Energieträgerstrom aus einer externen Energiequelle zugeführt wird.

3. Verfahren nach einem der vorhergehenden Ansprüche 1 oder 2, **dadurch gekennzeichnet, dass** eine Druck-Elektrolyseeinheit zur Erzeugung des Wasserstoffstroms (6) und des Sauerstoffstroms (7) vorgesehen wird, wobei der Wasserstoffdruck und/oder der Sauerstoffdruck zwischen 10 bis 200 bar beträgt.

4. Verfahren nach einem der vorhergehenden Ansprüche, **dadurch gekennzeichnet, dass** der Sauerstoffstrom (7) mit einem Speicherdruck von wenigstens 30 bar in einer Speichereinheit (23) gespeichert wird, wobei ein Sauerstoffstrom (24) der Speichereinheit (23) entnommen und auf dem Speicherdruck der Brennkammer (4) zugeführt wird.

5. Verfahren nach einem der vorhergehenden Ansprüche, **dadurch gekennzeichnet, dass** ein Kühlwasser- oder Kondensatstrom (25) aus der Reaktoreinheit (3) und/oder ein aus dem Rauchgasstrom (15) abgeschiedener Kondensatstrom (33) zur Brennkammer (4) geführt wird.

6. Verfahren nach einem der vorhergehenden Ansprüche, **dadurch gekennzeichnet, dass** ein Kühlwasser- oder Kondensatstrom (35) aus der Reaktoreinheit (3) und/oder ein aus dem Rauchgasstrom (15) abgeschiedener Kondensatstrom (33) zur Elektrolyseeinheit (2) geführt wird.

7. Verfahren nach einem der vorhergehenden Ansprüche, **dadurch gekennzeichnet, dass** bei der Erzeugung des Energieträgerstroms (13) in der Reaktoreinheit (3) freiwerdende Reaktionswärme aus der Reaktoreinheit (3) ausgekoppelt wird.

8. Energieträger-Erzeugungsanlage (1) mit einer Elektrolyseeinheit (2) ausgebildet zur Wasserstofferzeugung und Sauerstofferzeugung durch Elektrolyse eines wässrigen Mediums, mit einer Reaktoreinheit (3) ausgebildet zur Erzeugung eines kohlenwasserstoffhaltigen Energieträgerstroms (13) aus dem durch Elektrolyse erzeugten Wasserstoff und Kohlendioxid und mit einer Brennkammer (4) zur Verbrennung des kohlenwasserstoffhaltigen Energieträgerstroms (13) mit dem durch Elektrolyse erzeugten Sauerstoff, wobei der bei der Verbrennung gebildete Rauchgasstrom der Reaktoreinheit (3) als einzige Kohlenstoffquelle zuführbar ist, ausgebildet zur Durchführung des Verfahrens nach einem der vorhergehenden Ansprüche.

## Claims

1. A method of carbon dioxide-neutral compensation of energy peaks and valleys in the generation of electrical energy and/or for producing a fuel containing hydrocarbons, each using a fuel production system (1) in which electrical energy generated from an energy source is used in an electrolysis unit (2) of the fuel production plant (1) for producing hydrogen for electrolysis of a medium, in which a hydrocarbon stream (6) generated in the electrolysis unit (2) is supplied to a reactor unit (3) of the fuel production plant (1) for producing a fuel stream (13) containing hydrocarbons and using hydrogen and carbon dioxide, wherein the hydrocarbon-based fuel stream (13) thereby produced is burned at least partially and as needed with an oxygen stream (7, 24) produced in the electrolysis unit (2) in a combustion chamber (4) of the fuel production system (1), and the thermal energy of the exhaust gas stream (15) formed by the combustion is used to produce electrical energy in a gas turbine process and/or in a steam turbine process, and only the resulting exhaust gas stream (15) is supplied to the reactor unit (3) as the carbon source for producing the hydrocarbon-based fuel stream (13).

2. The method according to Claim 1, **characterized in that** the combustion chamber (4) receives at least one additional hydrocarbon-based fuel stream from an external energy source.

3. The method according to any one of the preceding Claims 1 or 2, **characterized in that** a pressure electrolysis unit is provided for generating the hydrogen stream (6) and the oxygen stream (7), wherein the hydrogen pressure and/or the oxygen pressure is between 10 and 200 bar.

4. The method according to any one of the preceding claims, **characterized in that** the oxygen stream (7) is stored with a storage pressure of at least 30 bar in a storage unit (23), wherein an oxygen stream (24) is withdrawn from the storage unit (23) and sent to the combustion chamber (4) at the storage pressure.

5. The method according to any one of the preceding claims, **characterized in that** a cooling water or condensate stream (25) from the reactor unit (3) and/or a condensate stream (33) condensed out of the exhaust gas stream (15) is sent to the combustion chamber (4).

6. The method according to any one of the preceding claims, **characterized in that** a cooling water or condensate stream (35) from the reactor unit (3) and/or a condensate stream (33) condensed out of the exhaust gas stream (15) is supplied to the electrolysis unit (2).

7. The method according to any one of the preceding claims, **characterized in that** in producing the fuel stream (13) in the reactor unit (3), the reaction heat thereby released is output from the reactor unit (3).

8. A fuel production plant (1) having an electrolysis unit (2) designed for generating hydrogen and generating oxygen by electrolysis of an aqueous medium, having a reactor unit (3) designed to generate a fuel stream (13) containing hydrocarbons from the hydrogen produced by electrolysis and the carbon dioxide and having a combustion chamber (4) for combustion of the hydrocarbon-based fuel stream (13) with the oxygen produced by electrolysis, wherein the exhaust gas stream formed by combustion can be supplied to the reactor unit (3) as the single carbon source, designed for performing the method according to any one of the preceding claims.

## Revendications

1. Procédé destiné à compenser, de façon neutre en dioxyde de carbone, des pointes et des baisses de production lors de la génération énergie électrique et/ou à produire une forme d'énergie contenant des hydrocarbures, dans les deux cas à l'aide d'une installation de production de formes d'énergie (1) dans laquelle on met en oeuvre de l'énergie électrique générée par une ressource énergétique pour produire, au sein d'une unité d'électrolyse (2) de l'installation de production de formes d'énergie (1), de l'hydrogène par électrolyse d'un milieu aqueux, un flux d'hydrogène (6) produit dans l'unité d'électrolyse (2) étant introduit dans une unité de réacteur (3) de l'installation de production de formes d'énergie (1) laquelle est réalisée de façon à produire un flux d'une forme d'énergie contenant des hydrocarbures (13) en mettant en oeuvre de l'hydrogène et du dioxyde de carbone, le flux d'une forme d'énergie contenant des hydrocarbures (13) étant, au moins partiellement et en fonction des besoins, brûlé au sein d'une chambre de combustion (4) de l'installation de production de formes d'énergie (1) avec un flux d'oxygène (7, 24) produit dans l'unité d'électrolyse (2), et l'énergie thermique contenue dans le flux de gaz de combustion (15) formé lors de la combustion étant utilisée pour générer de l'énergie électrique dans un processus à turbine à gaz et/ou un procédé à turbine à vapeur, et le flux de gaz de combustion (15) formé étant la seule source de carbone qui soit introduite dans l'unité de réacteur (3) pour produire le flux d'une forme d'énergie contenant des hydrocarbures (13).

2. Procédé selon la revendication 1, **caractérisé en ce qu'**au moins un autre flux d'une forme d'énergie contenant des hydrocarbures, issu d'une ressource énergétique externe, est introduit dans la chambre de combustion (4).

3. Procédé selon l'une des revendications 1 ou 2 précédentes, **caractérisé en ce que** l'on met en oeuvre une unité d'électrolyse sous pression destinée à produire le flux d'hydrogène (6) et le flux d'oxygène (7), la pression d'hydrogène et/ou la pression d'oxygène étant comprise(s) entre 10 et 200 bar.

4. Procédé selon l'une des revendications précédentes, **caractérisé en ce que** le flux d'oxygène (7) est stocké sous une pression de stockage d'au moins 30 bar dans une unité de stockage (23), un flux d'oxygène (24) étant prélevé dans l'unité de stockage (23) et introduit dans la chambre de combustion (4) en maintenant la pression de stockage.

5. Procédé selon l'une des revendications précédentes, **caractérisé en ce qu'**un flux d'eau de refroidissement ou de condensat (25) issu de l'unité de réacteur (3) et/ou un flux de condensat (33) séparé du flux de gaz de combustion (15) est/sont acheminé(s) vers la chambre de combustion (4).

6. Procédé selon l'une des revendications précédentes, **caractérisé en ce qu'**un flux d'eau de refroidissement ou de condensat (35) issu de l'unité de réacteur (3) et/ou un flux de condensat (33) séparé du flux de gaz de combustion (15) est/sont acheminé(s) vers l'unité d'électrolyse (2).

7. Procédé selon l'une des revendications précédentes, **caractérisé en ce que** la chaleur de réaction libérée lors de la production du flux d'une forme d'énergie (13) dans l'unité de réacteur (3) est extraite de l'unité de réacteur (3).

8. Installation de production de formes d'énergie (1), comportant une unité d'électrolyse (2) réalisée de façon à produire de l'hydrogène et de l'oxygène par électrolyse d'un milieu aqueux, une unité de réacteur (3) réalisée de façon à produire un flux d'une forme d'énergie contenant des hydrocarbures (13) à partir de l'hydrogène produit par électrolyse et de dioxyde de carbone, et une chambre de combustion (4) destinée à brûler le flux d'une forme d'énergie contenant des hydrocarbures (13) avec l'oxygène produit par électrolyse, le flux de gaz de combustion formé lors de la combustion pouvant être la seule source de carbone qui soit introduite dans l'unité de réacteur (3), réalisée de façon à pouvoir mettre en oeuvre le procédé selon l'une des revendications précédentes.
